(19) Europäisches Patentamt
European Patent Office
Office européen des brevets

(11) **EP 1 233 940 B1**

(12) **EUROPEAN PATENT SPECIFICATION**

(45) Date of publication and mention
of the grant of the patent:
**11.05.2005 Bulletin 2005/19**

(51) Int Cl.7: **C07C 53/16**

(86) International application number:
**PCT/EP2000/011788**

(21) Application number: **00977577.6**

(22) Date of filing: **23.11.2000**

(87) International publication number:
**WO 2001/040160 (07.06.2001 Gazette 2001/23)**

(54) **MONOCHLOROACETIC ACID GRANULES**

MONOCHLORESSIGSÄURE-GRANULATE

GRANULES D'ACIDE MONOCHLOROACETIQUE

(84) Designated Contracting States:
**AT BE CH CY DE DK ES FI FR GB GR IE IT LI LU
MC NL PT SE TR**

(30) Priority: **01.12.1999 EP 99204066**

(43) Date of publication of application:
**28.08.2002 Bulletin 2002/35**

(73) Proprietor: **Akzo Nobel N.V.
6824 BM Arnhem (NL)**

(72) Inventor: **PRIES, Willem
7231 NT Warnsveld (NL)**

(74) Representative: **Schalkwijk, Pieter Cornelis et al
Akzo Nobel N.V.
Intellectual Property Department
P.O. Box 9300
6800 SB Arnhem (NL)**

(56) References cited:
**EP-A- 0 693 472          GB-A- 1 468 790
US-A- 4 383 121**

- **CHEMICAL ABSTRACTS, vol. 91, no. 11, 10
September 1979 (1979-09-10) Columbus, Ohio,
US; abstract no. 91176e, B. EGLUND: "Dry
sodium monochloroacetate by neutralizing
monochloroacetic acid with alkali and drying"
page 736; column 1; XP002136447 & SE 407 797
A 23 April 1979 (1979-04-23)**
- **"Catalogue Handbook of Fine Chemicals" 1988,
ALDRICH**

## Description

The present invention pertains to a granulate form of monochloroacetic acid and to a process for providing such.

Monochloroacetic acid (MCA) is an important industrial chemical which is widely used in the preparation of pharmaceutical and agricultural products.

Monochloroacetate is mainly supplied in the form of flakes, which are made by cooling MCA liquid to below its melting point (62°C) on a flaker belt. At the end of the flaker belt, the flakes fall into flexible intermediate bulk containers (FIBCs) having a typical volume of 0.5-2 $m^3$, in which they are further stored and transported. These containers, usually in the form of bags, typically weigh about 1,000 kg when packed with MCA flakes.

In US 4,383,121 monochloroacetic acid grains were obtained by crystallization, but no granules thereof were made.

A problem with such flakes is that a large amount of MCA dust (fines) results on packing and emptying of these big bags, which is hazardous, since MCA is irritating and corrosive to the skin.

Furthermore, the form of the MCA solid reduces its flowability. Moreover, MCA flakes have a tendency to cake, i.e. clump together, during transportation. Their flat shape and the fines between the flakes create a large contact area, which promotes bud formation, resulting in a caked product. This is particularly problematic when the MCA flakes are transported in FIBCs under conditions of high temperature and high humidity. Under such conditions, the MCA flake surface becomes particularly weak and sticky.

An object of the present invention is to overcome one or more of the above problems associated with MCA flakes.

The present invention provides monochloroacetic acid characterized in that the compound is in the form of a granule, said granule having a diameter in the range of 2-10 mm and a diameter to length ratio of 0.05-2, preferably 0.25-1.

The granules according to the present invention have a well-defined shape and a substantially uniform granule size distribution.

The weight percentage of fines (dust particles smaller than, for example, about 50% of the diameter of the granules) associated with the granules according to the present invention was measured by the inventors to be less than 10 wt. %, and usually less than 5 wt.% of the granules, which is substantially less than the amount of fines associated with MCA flakes.

The bulk density of the granulate according to the present invention lies in the range between 700-1,000 kg/ $m^3$, preferably in the range of 800-900 kg/$m^3$.

The bulk density of the granulate according to the present invention was measured to be approximately 1000 kg/$m^3$, which is higher than the bulk density of MCA flakes.

The granules according to the present invention preferably have a solubility rate to an about 50% aqueous solution, measured at a stirrer speed of about 120 rpm and about 20°C of at least about 0.20 g/s, preferably at least about 0.25 g/s.

The solubility rate can also be determined in isopropyl alcohol rather than water.

The solubility rate in isopropyl alcohol to an about 5% solution, measured at about 120 rpm and about 20°C is at least about 35 mg/s, preferably at least about 45 mg/s.

The granules according to the present invention accordingly exhibit a solubility performance in industrial applications corresponding to that of MCA flakes.

The granules preferably have a caking strength of less than 0.10, more preferably of less than 0.05 kg/$cm^2$.

Granules according to the present invention exhibit a caking strength which is a factor of about 2-3 lower than the caking strength of flakes, with the granules having less tendency to clump together.

According to a second aspect of the invention, there is provided a composition of matter consisting of a holder, preferably an FIBC, containing a predetermined amount of the granules according to the invention, wherein the weight percentage of fines is 10 or less, preferably 5 or less. Packing and transporting the granules in such a holder leads to a transportable and storable product having less tendency to clump than MCA flakes under extreme weather conditions, and where there is less risk of the user coming into contact with skin irritating fines during packing and unpacking of the granules.

According to a third aspect of the present invention, a process for producing these granules is provided which comprises the steps of crushing flakes of monochloroacetic acid, with the flakes being heated to near their melting points, followed by pressing the so crushed flakes through a die plate the hole diameter of which is in the range of 5-7 mm, and the hole depth of which is in the range of 20-80, preferably 50-60 mm. The flakes are subjected to a pressure of 4-7, preferably 5-6 MPa.

Re-shaping MCA flakes to MCA granulate according to this process provides well-defined granules having a substantially uniform size distribution.

The process is preferably carried out substantially in the absence of lubricants and binders, since friction between the compressed granules and the walls of the die heats the outside of the granules to their melting temperature, whereby a lubricant action is provided. Accordingly, the provision of expensive lubricants and binders is unnecessary, the purity is improved, and there is no need for process steps for removing added lubricants from the final granules.

Via the process an MCA granule can be obtained of which at least about 80% of the particles is between the 3.15 and 8 mm and where less than about 5% is below about 3.15 mm, without product screening.

The invention will be further described by way of the following description, which refers to the accompanying figures wherein:

-    figure 1 is a schematic route showing the apparatus used for providing MCA granules from MCA flakes;
-    figure 2 is a graph showing the relation among the thickness of the die plate of the granulating machine and the granule temperature and the percentage of fines;
-    figure 3 shows the production rate in kg/h of granules made via the process according to the present invention.
-    figure 4 schematically shows a hole in a die plate of the granulating machine;
-    figures 5A and B show apparatus used for determining the bulk density and the tapped bulk density of MCA;
-    figure 6 shows apparatus used for investigating the slit width and the angle of repose of MCA granules; and
-    figure 7 shows apparatus used for investigating the caking strength of MCA granules.

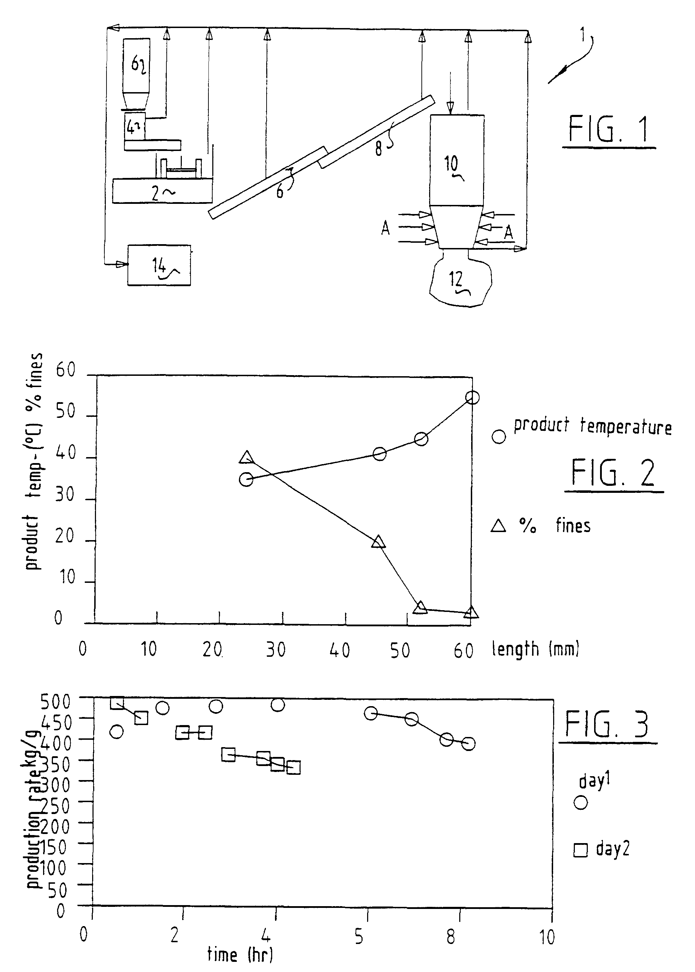

A pilot plant 1 was constructed around a granulator with exchangeable die plates (figure 1). MCA flakes were fed into the granulator via a screw feeder 4, loaded from a hopper 6 of capacity 1 m$^3$. Granules produced by the granulator 2 were transported by two belt conveyors 6, 8 into a 2 m$^3$ silo 10. The silo 10 was aerated with compressed air to cool the granules, in order to minimize caking. The granules from the silo 10 were discharged via a rotary valve (not shown) into FIBCs. After the test trials, a portion of the granules was sieved with a vibrating sieve to remove dust < 3 mm from the product.

Sampling and analyzing

-    A sample of approx. 25 liters of the MCA granules was collected at the overflow of the belt conveyor 8 into the cooler/silo 10.
-    The following analyses were carried out on the collected samples:

    .    Capacity of the granulation machine (stopwatch, weighing balance)
    .    Product temperature (thermometer)
    .    Particle size distribution (sieve analysis)
    .    Solubility rate (time in water, and/or isopropyl alcohol)
    .    Bulk density, tapped bulk density (defined pouring into container with exactly known volume)
    .    Angle of repose
    .    Slit width
    .    Caking test (exposure of granules to several climatological and physical conditions).

Investigating the effect of different die plates on the MCA granules

Table 1 shows the effect of the different die plates on the quality of the MCA granules.

## Table 1 Production of MCA granulate

| | | Die (mm/mm) | | | | | | |
|---|---|---|---|---|---|---|---|---|
| | | 4/8 | 5/20 | 6/24 | 6/48 | 6/54 | 6/60 | 6/60 |
| | | 87 rpm | 105 rpm | 87 rpm | 87 rpm | 87 rpm | 87 rpm | 105 rpm |
| pressure ($10^5$ Pa) | | 50-60 | 50-60 | 50-60 | 50-60 | 50-55 | 50-55 | 50-55 |
| product temp. (°C) | | 31 | 45 | 35 | 42 | 45 | 55 | 55 |
| fines/dust (%<3.15 mm) | | 50 | 17 | 40 | 20 | 1-5 | 1-3 | 1-5 |
| Production rate (kg/h) | | | | | | 500-600 | 500-600 | 700-800 |

The dimensions of the die holes, e.g. 6/20 (mm/mm) = diameter/depth

The tests with die plate diameters/depths of 4 mm/8 mm, 5 mm/20 mm, and 6 mm/24 mm showed that a 6 mm diameter product had the best visual performance. However, the quantity of fines was too high.

The granules obtained from the 6 mm/60 mm plate showed a high temperature (55°C) during a longer production run. The (uncooled) granules had a very severe caking tendency.

A 6 mm/48 mm die plate gave an amount of fines.

A 6 mm/54 mm die plate yielded better results, a low percentage of fines and a moderate product temperature (45°C). The tests with the die plates gave a model for the granule formation. The forced transport through the holes of the die plate raised the temperature of the MCA at the wall up to or above the softening temperature (>45°C). The soft MCA was easy to pelletize. The outside of the granule was partly molten MCA and cooled down immediately after leaving the die plate. When the hole depth was too low, the friction energy was too low and the softening, melting temperature at the wall could not be reached. The resulting MCA had too many fines.

When the hole depth was large (see the 6 mm/60 mm die plate), the friction caused a high temperature, which led to a softer product with a higher caking tendency.

Figure 2 shows the relation among the thickness of the die plate and the product temperature and the % fines for the 6 mm holes.

Figure 3 shows the production rate of MCA granules in a granulator using a 6 mm/54 mm die plate.

The product temperature increased linearly with the compacting length. Above a compacting length of 54 mm, the temperature rose more than proportionally. An explanation for this may be the higher energy input/ton product for the 60 mm plate compared with the 54 mm die plate.

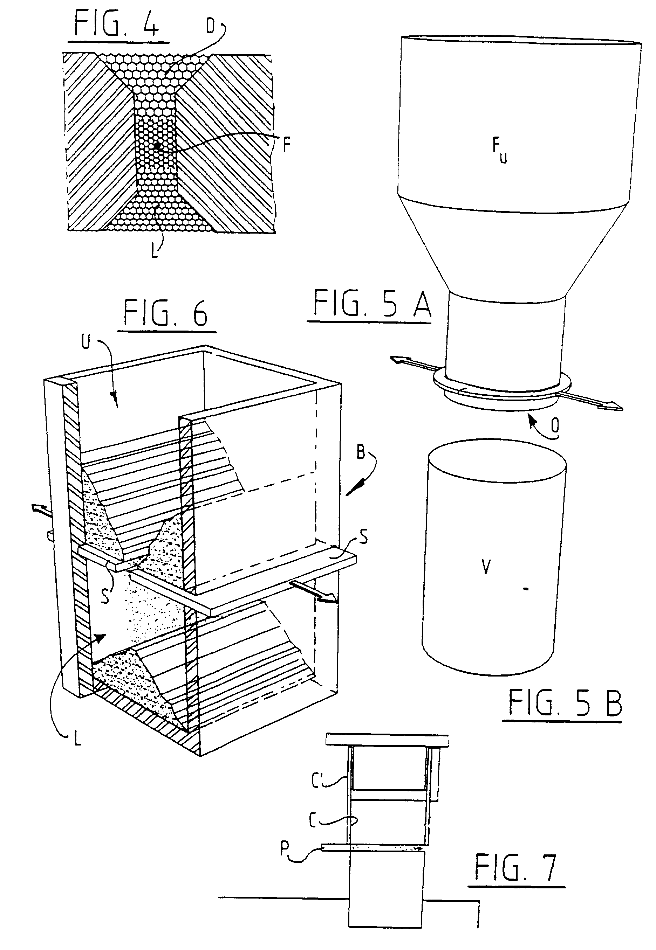

Figure 4 schematically shows a hole in a die plate. In the first densification zone, the MCA is densified. The wall friction, caused by the transport through the hole, results in a rise of the temperature. This is called the second friction zone. The third lubrication zone begins at the softening point of the MCA. Here, the MCA is gliding through the hole. After leaving the hole, the softened outside of the granulate cools and is hardened. The inner, unsoftened part of the granule is encapsulated.

Effect of roller speed of the granulator

Table 1 also shows the influence of the roller speed (last column). Increasing the roller speed (at the 6 mm/60 mm die plate) from 87 to 105 rpm resulted in a 33% increase of the production rate.

A test at a roller speed of 130 rpm (6 mm/60 mm plate) resulted in more dust and a softer product. The MCA granules obtained in tests with a 48 mm die plate showed a similar behavior. A possible explanation of this phenomenon is that the residence time in the holes was so short that only the densification and the friction zone occurred and that there was no lubrication zone. There was no encapsulation of the unsoftened product but only of the densified product.

Determination of the rate of dissolution of MCA flakes, granules according to the present invention, compacted and other particles >1.5 mm in water, was carried out as follows.

Apparatus and equipment

Stirring motor, 0 - 250 rpm
Mixing paddle, width of the paddle is 3/4 of the diameter of the mixing beaker (3.2)
Beaker, glass, volume 500 ml.
Heating plate, adjustable at 20-80°C
Balance, scale reading 0.01 g.
Stopwatch, scale reading 1 sec.
Temperature meter, scale reading 1°C

Procedure

300 ml water were added to the 500 ml beaker. The beaker was placed on the heating plate and the temperature of the water was adjusted to 20°C. The water was mixed using the paddle.
300 g (= Ms g) of the MCA sample were added and dissolution was timed.
The rate of dissolution (S) was calculated using the formula:

$$S = Ms \, / \, t \text{ (g/second)}$$

wherein:

$Ms$ = mass in g, of the sample
$t$ = time for complete dissolution of the sample, in seconds

Table 2 shows the results.

Table 2.

| Product performance of several MCA products | | | | | | |
|---|---|---|---|---|---|---|
| Solubility rate | | Flakes | Granulate | | Granulate pilot production Kahl6mm | Compacts Lab machine Bepex Cigars | Pastilles hand made |
| | | | lab machine Kahl | | | | |
| | | | 2 mm | 5 mm | 6 mm | | |
| Water | | | | | | | |
| 50% solution (g/s) | | | | | | | |
| 20 rpm | 20°C | 0.29 | | 0.29 | 0.27 | | |
| 200 rpm | 20°C | 0.55-0.65 | 1.30 | 0.55 | 0.45-0.65 | 0.41 | 0.97 |
| IPA | | | | | | | |
| 5% solution | | | | | | | |
| 120 rpm | 20°C | 35-40 | | 50 | 45 | | |
| 200 rpm | 20°C | 60-65 | | | 52-60 | | |
| Caking strength (kg/cm$^2$)* | | 0.06-0.13 | | 0.06 | 0.00-0.04 | n.m. | n.m. |

*This is the range of values of several measurements

The caking conditions were: 6h 30°C/30%RH; 6h 20°C/65%RH. The test lasted 3 days

Table 2 shows that the solubility rate decreased with increasing size. The solubility rate was determined by the surface area and the stirrer speed. 5 and 6 mm granules have approximately the same solubility rate as flakes.

Determination of the poured and the tapped bulk density of MCA flakes, granules, compacts, and other particles > 1.5 mm

Apparatus and equipment (figure 5A, 5B)

-    Balance, scale reading 1 g.
-    Funnel $F_u$ (stainless steel) and transparent calibrated vessel V. The calibration was a linear tape with a reading of 0.1 cm. The volume of the vessel was 13.7 liters.

Procedure

The mass of the empty vessel rounded-off in grams (M0 g) was determined first. The discharge opening O of the funnel was closed, and the funnel was filled with 14-15 liters of the solid MCA product. The vessel was centered under the discharge opening. The discharge opening was opened and material allowed to flow into the Perspex™ vessel until this was filled. The excess of material was scraped off. The mass of the vessel with product (M1 g) was determined. The poured bulk density was calculated with the formula:

$$\text{poured bulk density} = \frac{M_1 - M_0}{13.7} \text{(g/L) or (kg/m}^3)$$

The vessel with product was lifted and tapped 10 times on the ground.
The tapped bulk density was calculated with the following formula:

$$\text{tapped bulk density} = \frac{(M_1 - M_0)}{V} \text{(g/l)(kg/m}^3)$$

wherein:

$M_1$ = mass vessel + material
$M_0$ = mass vessel
V = volume MCA in vessel. This is calculated with the formula:

$$V = \frac{h}{34.5} \times 13.7$$

h = height of MCA level (cm)
34.5 = height of vessel (cm)
13.7 = volume of vessel (l)

A higher tapped bulk density enables more MCA to be packed into the same volume.
The ratio between the "tapped" and the "poured" density is a measure of the settling degree or settling ability of the MCA in an FIBC for example. and is called the "Hausner" ratio. The lowest possible value is 1.00 and applies for fully free-flowing materials (such as glass spheres). At 1.20 the materials become semi-cohesive, and materials with values higher than 1.40 are cohesive (talc powder). In general it can be stated that the higher the ratio, the worse the flowability of the material.

Determination of the angle of repose of monochloroacetic acid flakes. granules, compacts, and other particles.

Principle

Pouring MCA flakes, granules, compacts in a heap in a controlled way. The samples were poured into a transparent rectangular box B provided with a slit halfway down (figure 6). The angle of the heap with the horizontal plane was measured. The angle of repose is a measure of flowability. Generally spoken, the lower the angle, the better the flowability.

Procedure

The slides S of the apparatus were closed. The upper part of the apparatus was filled with 18 - 24 liters of the solid MCA product.
The slides S were slowly opened and stopped when the material started to flow and pour onto the bottom of the box. The test ended when no more material flowed through the slides. The angle of repose of the poured MCA was determined by measuring the angle of the formed heap (with the horizontal plane) with a protractor.

Determination of the slit width of monochloroacetic acid (MCA) flakes, granules, compacts, and other particles >1.5 mm.

Principle

To investigate the flow of MCA flakes, granules, compacts from a hopper with an adjustable slit width to provide a controllable flow were used. The hopper was the same transparent rectangular box as used for measuring the angle of repose. The slit width at which the solid MCA started to flow was measured. The slit width is a measure of flowability. The lower the slit width, the better the flowability in general.

Apparatus and equipment

A transparent Perspex™ rectangular box B of 300 mm in length and 300 mm in width was used (as also shown in figure 6).

Procedure

The slits S of the box were closed. The upper part U of the box was filled with 18 - 24 liters of the solid MCA product.
The slits were opened.
The test ended when no more material flowed through the slits into the lower part L of the box B. The slit width was determined with a ruler by measuring the gap between the two slits.

Product performance

Table 3 shows the results of the performance tests of different MCA products.

Table 3. Performance of several MCA shapes

| | flakes | granules lab machine Kahl | | granulate pilot production Kahl 6mm/54mm | compacts lab machine Bepex Cigars | Pastilles hand made |
|---|---|---|---|---|---|---|
| | | 2 mm | 5 mm | | | |
| bulk density (kg/m³) | 720 | 760 | 820 | 850 | 720 | n.m. |
| tapped bulk density (kg/m³) | 820 | 810 | 910 | 965 | 790 | n.m. |
| Hausner ratio | 1.14 | 1.07 | 1.11 | 1.13 | 1.10 | n.m. |
| Slit width (mm) | 30 | 15 | 30 | 18 | 125 | n.m. |
| angle of repose | 31 | 41 | 31 | 28 | 31 | n.m. |

Results

The performance of the granules is dependent on the diameter of the granules. A greater diameter resulted in higher bulk and tapped bulk densities and a lower angle of repose. The flowability of 6 mm granules is better than the flowability of flakes.

Chemical quality

The chemical quality of MCA was determined by the quality of the incoming flakes and the chemical pick-up of metals in the granulation process.
The Fe content of the incoming MCA mostly varied between 1 and 5 ppm. The compacting properties of the flakes were better when the flakes were fresher.
The Fe content of the outcoming MCA granules varied between 6 and 19 ppm, where the higher Fe contents of the granules correspond with the high Fe content of the flakes.

Particle size distribution

Table 4 shows the particle size distributions of granules and Table 5 of the flakes originating from the pilot plant. The particle size distribution was measured with a vibrating sieve stack.

Table 4.

| Particle size distribution of granules and flakes | | | | | | |
|---|---|---|---|---|---|---|
| Test h Product | 0 granules | 1 granules | 2.25 granules | 3.5 granules | 5.25 granules | 7.25 granules |
| <3.15 mm | 1 | 3 | 4 | 2 | | 1 |
| 3.15-6.3 mm | 43 | 59 | 63 | 61 | 71 | 70 |
| 6.3-8.0 mm | 43 | 33 | 26 | 32 | 17 | 27 |
| >8.0 mm | 14 | 6 | 7 | 4 | 1 | 2 |

Table 5.

| Particle size distribution of flakes from two different big bags | | |
|---|---|---|
| Product | flakes ex big bag 1 | flakes ex big bag 2 |
| <3.15 mm | 28 | 14 |
| 3.15-6.3 mm | 34 | 33 |
| 6.3-8.0 mm | 16 | 26 |
| >8.0 mm | 22 | 27 |

Determination of caking properties of monochloroacetic acid

Principle of measurement

The powder is stored under well-defined temperature and relative humidity conditions for a given time under a prescribed pressure. After this consolidation period, the powder will have formed a compact with a certain strength. This strength is called "caking strength" and is characterized by the force necessary to break the compact.

Equipment

Figure 7 shows the cake tester.
The test conditions are chosen in relation to the actual storage conditions that are simulated. Table 6 gives the measure of the cake tester.

Table 6

| No. | Material | Diameter D (mm) | Height H (mm) | Area cm$^2$ | Volume cm$^3$ | Product particle sizes | Test conditions (temp. and RH) |
|---|---|---|---|---|---|---|---|
| 1 | PVC | 155 | 150 | 189 | 2830 | >6mm | constant |

PVC is not corroded by MCA, whereby undesirable wall effects, adhering of MCA to the PVC, are limited.

Conditioning of MCA sample

The samples for testing should be homogeneous.
The sample must be conditioned for at least 24 hours under the (starting) conditions of the cake test (temperature and relative humidity). For a standard test these conditions are: temperature $20 \pm 2°C$ and relative humidity $50 \pm 5$ %.

Procedure

For the MCA standard, the test conditions are alternating 6 h at 30°C/30% RH and 6 h at 20°C/65% RH during 3 days (RH = relative humidity).

A. Preparation

The consolidation load F is 7 kg (= 0.04 kg/cm$^2$)
The weights needed to realize the consolidation load F were arranged, taking the weight of the cover plate into consideration also. The weight of this cover plate also contributes to the consolidation but is small compared with the 7 kg load.
The cake tester was cleaned.

B. Filling of the cake tester

The pin P was put into the inner cylinder C and the outer cylinder C' was slid over the inner cylinder C until it rested on the pin.
The funnel was filled with conditioned sample.

The funnel was slowly raised, allowing the sample to gradually fill the cake tester.

The cake tester was filled completely.

The inner cylinder C was held. The outer cylinder C' was tapped three times with the cover plate to settle the sample.

The excess sample was scraped off, so that the sample surface was level with the top of the outer cylinder C'.

The cover plate was placed on the outer cylinder C'.

The cake tester was put on its base plate under storage conditions, it being ensured that the cake tester was in a stable and vertical position.

The weights were loaded.

The outer cylinder was held in a fixed position and the pin was removed.

The outer cylinder was released, allowing the sample to be compressed under the consolidation weight.

### C. Measurement of caking strength by applying weights

At the end of the consolidation period of 3 days, the weights were removed along with the cover plate.

The outer cylinder was slid down until it cleared the MCA compact completely.

The cover plate was placed on the compact.

The load on the MCA compact was increased by loading weights in steps of 0.5 kg on the cover plate until the compact crumbled. The total load (kg) needed to break the compact ($F_c$) was calculated.

The caking strength of granules, the force needed to crush a caked sample of a defined shape, was considerably lower than the caking strength of flakes. The caking strength was also influenced by the particle size distribution. The more the particles are in contact with each other, the higher the caking strength. Table 7 shows the influence of fines on the caking strength.

### Table 7. Influence of fines on the caking strength

| | flakes with fines | flakes <3.15 mm | granules with fines | granules <3.15 mm |
|---|---|---|---|---|
| caking strength ($kg/m^3$) | 0.06-0.13 | 0.06-0.010 | 0.03-0.05 | 0.00-0.04 |

### Claims

1. A granule of monochloroacetic acid wherein said granule has a diameter in the range of 2-10 mm and a diameter to length ratio of 0.05-2.

2. A granule of claim 1 wherein the diameter to length ratio is 0.25-1.

3. A granule of claim 1 or 2 wherein the bulk density lies in the range of 700-1,000 $kg/m^3$.

4. A granule of claim 3 wherein the bulk density lies in the range of 800-900 $kg/m^3$.

5. A granule of any one of claims 1-4 having a solubility rate to an about 50% aqueous solution measured at 120 rpm and 20°C of at least 0.20 g/s, preferably at least 0.25 g/s.

6. A granule of any one of claims 1-5 having a caking strength of less than 0.10, preferably less than 0.05 $kg/m^3$.

7. A composition of matter consisting of a holder and the granules of any one of claims 1-6, wherein the weight percentage of fines is 10 or less, preferably 5 or less.

8. A process for providing granules according to any one of claims 1-6, comprising the steps of crushing flakes of monochloracetic acid with the flakes being heated to near their melting points, followed by pressing the so crushed flakes through a die plate the hole diameter of which is in the range of 5-7 mm, and the hole depth of which is in the range of 20-80, preferably, 50-60 mm.

9. The process according to claim 8 wherein the process is carried out substantially in the absence of lubricants and binders.

10. The process according to claim 8 or 9 wherein the flakes are subjected to a pressure of 4-7, and preferably 5-6 MPa.

**Patentansprüche**

1. Monochloressigsäure-Körnchen, wobei das Körnchen einen Durchmesser im Bereich von 2 bis 10 mm und ein Verhältnis von Durchmesser zu Länge von 0,05 bis 2 hat.

2. Körnchen gemäß Anspruch 1, wobei das Verhältnis von Durchmesser zu Länge von 0,25 bis 1 ist.

3. Körnchen gemäß den Ansprüchen 1 oder 2, wobei die Schüttdichte im Bereich von 700 bis 1000 kg/m$^3$ liegt.

4. Körnchen gemäß Anspruch 3, wobei die Schüttdichte im Bereich von 800 bis 900 kg/m$^3$ liegt.

5. Körnchen gemäß irgendeinem der Ansprüche 1 oder 4, das eine Löslichkeitsrate gegenüber einer etwa 50%igen wässrigen Lösung, gemessen bei 120 U/min und 20 °C, von wenigstens 0,20 g/s, vorzugsweise von wenigstens 0,25 g/s hat.

6. Körnchen gemäß irgendeinem der Ansprüche 1 oder 5, das ein Backvermögen von weniger als 0,10 kg/m$^3$, vorzugsweise von weniger als 0,05 kg/m$^3$ aufweist.

7. Material-Zusammensetzung, die aus einer Haltevorrichtung und den Körnchen gemäß irgendeinem der Ansprüche 1 bis 6 besteht, wobei der Gewichtsprozentgehalt an Feinstkorn 10 oder weniger, vorzugsweise 5 oder weniger ist.

8. Verfahren zum Bereitstellen von Körnchen gemäß irgendeinem der Ansprüche 1 bis 6, umfassend die Schritte des Zerkleinerns von Monochloressigsäure-Plättchen, wobei die Plättchen nahe an ihre Schmelzpunkte heran erwärmt werden, und des anschließenden Pressens der so zerkleinerten Plättchen durch eine Düsenplatte, deren Lochdurchmesser im Bereich von 5 bis 7 mm liegt und deren Lochtiefe im Bereich von 20 bis 80 mm, vorzugsweise von 50 bis 60 mm liegt.

9. Verfahren gemäß Anspruch 8, wobei das Verfahren im Wesentlichen in Abwesenheit von Gleitmitteln und Bindemitteln durchgeführt wird.

10. Verfahren gemäß den Ansprüchen 8 oder 9, wobei die Plättchen einem Druck von 4 bis 7 MPa und vorzugsweise von 5 bis 6 MPa ausgesetzt werden.

**Revendications**

1. Granule d'acide monochloroacétique où ledit granule a un diamètre dans la plage de 2 à 10 mm et un rapport du diamètre à la longueur de 0,05 à 2.

2. Granule selon la revendication 1, où le rapport du diamètre à la longueur est de 0,25 à 1.

3. Granule selon la revendication 1 ou 2, où la densité apparente est dans la plage de 700 à 1 000 kg/m$^3$.

4. Granule selon la revendication 3, où la densité apparente est dans la plage de 800 à 900 kg/m$^3$.

5. Granule selon l'une quelconque des revendications 1 à 4 ayant un taux de solubilité dans une solution aqueuse à 50 %, mesuré à 120 tr/min et à 20 °C, d'au moins 0,20 g/s, de préférence d'au moins 0,25 g/s.

6. Granule selon l'une quelconque des revendications 1 à 5 ayant une force agglomérante inférieure à 0,10, de préférence inférieure à 0,05 kg/m$^3$.

**7.** Composition d'une matière constituée d'un support et les granules selon l'une quelconque des revendications 1 à 6, dans laquelle le pourcentage en poids des fines est de 10 ou moins, de préférence de 5 ou moins.

**8.** Procédé pour fournir des granules selon l'une quelconque des revendications 1 à 6, comprenant les étapes consistant à écraser des flocons d'acide monochloroacétique, les flocons étant chauffés aux environs de leur point de fusion ; puis à presser les flocons ainsi écrasés au travers d'un plateau de moulage dont le diamètre des trous est dans la plage de 5 à 7 mm, et la profondeur des trous est dans la plage de 20 à 80, de préférence de 50 à 60 mm.

**9.** Procédé selon la revendication 8 où le processus est réalisé sensiblement en l'absence de lubrifiants et de liants.

**10.** Procédé selon la revendication 8 ou 9 dans lequel les flocons sont soumis à une pression de 4 à 7, et de préférence de 5 à 6 MPa.

FIG. 1

○ product temperature

FIG. 2

△ % fines

FIG. 3

day1
○

□ day2

FIG. 4

FIG. 5 A

FIG. 6

FIG. 5 B

FIG. 7